# EUROPEAN PATENT APPLICATION

(11) **EP 4 796 169 A1**
(43) Date of publication of application: **26.08.2026**
(21) Application number: 25159064.2
(22) Date of filing: 20.02.2025
(51) Int. Cl.: A61N 5/10

(54) **RADIATION TREATMENT PLANNING FOR SPATIALLY FRACTIONATED RADIOTHERAPY**

(71) Applicant: Siemens Healthineers International AG, 6312 Steinhausen (CH)
(72) Inventor: KUUSELA, Esa, 02320 Espoo (FI)
(74) Representative: Mathisen & Macara LLP

(57) **Abstract**

A method (700), computer system (100) and computer program product for generating and optimising a radiotherapy treatment plan for spatially fractionated radiotherapy, the method (700) comprising: constructing (704, 706) a model (400) of patient anatomy, the model comprising a target volume (420) and a plurality of sub-targets (450), wherein the sub-targets (450) are determined by dividing the target volume (420); and generating the treatment plan by simultaneously optimising (708) a combination of both (i) dose objectives and (ii) sub-targets characteristics.

## Description

### TECHNCAL FIELD

This invention relates to radiation therapy treatment planning, and in particular, to methods and systems for generating and optimising treatment plans for spatially fractionated radiotherapy.

### BACKGROUND

### Radiotherapy

The use of energy to treat medical conditions comprises a known area of prior art endeavour. For example, radiation therapy comprises an important component of many treatment plans for reducing or eliminating unwanted tumours. Unfortunately, applied energy does not inherently discriminate between unwanted material and adjacent tissues, organs, or the like that are desired or even critical to continued survival of the patient (such adjacent tissues may be referred to as "organs at risk", OARs). As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform "machine" parameters during each of a plurality of sequential fields. For example, machine parameters of a radiotherapy system may include the angle of irradiation of the treatment beam, or the configuration of the leaves of a multileaf collimator.

### Plan optimisation using cost functions

Treatment plans for radiation treatment sessions are often automatically generated through a so-called optimisation process. As used herein, "optimisation" will be understood to refer to improving a candidate treatment plan without necessarily ensuring that the optimised result is, in fact, the singular best solution. Such optimisation often includes automatically adjusting one or more physical treatment machine parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

In some cases, optimisation proceeds as a function of multiple different criteria. This may involve the use of a cost function comprising a mathematical model that attempts to balance conflicting clinical goals to produce a treatment plan with the least "cost". Clinical goals generally take the form of a particular metric being required to be greater or smaller than a threshold value. For example, a clinical goal may require that a certain percentage of the volume of an OAR receives less than a certain amount of dose. As a further example, a clinical goal may require that a certain percentage of the planning target volume (PTV) receives at least a certain amount of dose. A cost function may be a sum of terms relating to deviations from the clinical goals.

As an alternative to a cost function, a utility function may be used. Utility functions have terms that reward adherence to clinical goals, and wherein said optimisation comprises maximising the utility function; and wherein the utility function may further contain a reward term that guides the optimisation. In general, in the present disclosure, a "utility function" may be used instead of a "cost function", wherein the optimiser attempts to maximise the utility of the utility function instead of minimising the cost of the cost function.

A computer-implemented optimiser is generally used to iteratively maximise (or minimise) the utility function (or cost function). In general, the process of optimisation involves finding a set of machine parameters (also referred to as "control points") that maximise a given utility function (or minimise the cost function).

### Spatially fractionated radiotherapy

Recently, a radiotherapy method referred to as "spatially fractionated radiotherapy" (SFRT) has gained interest from cancer treatment professionals. The fundamental idea behind this method is the construction of a model of patient anatomy, the model comprising a target volume and a plurality of sub-targets. The sub-targets are determined by dividing the target volume. Thus, the target volume is divided (or "spatially fractionated") into sub-targets.

The sub-targets are typically spherical or cylindrical in shape, although other shapes are possible.

The prescription dose for each sub-target is the same as the original prescription dose for the overall target volume in the region occupied by the sub-target. In general, the prescription dose for the regions in between the sub-targets is planned to be as small as reasonably possible. That is, there is a "quick fall-off" in prescription dose outside the sub-targets as compared to the prescription dose for inside the sub-target. This is to simplify the planning process and have more control over the radiation delivered.

### Cost functions in SFRT

Traditionally, a utility function or cost function is developed on the basis of a desired dose distribution. For example, a standard optimisation scheme may be used to generate a set of control points, machine parameters and MLC leaf patterns that, ideally, simultaneously produce adequate dose distribution to the target region, whilst also maintaining the dose in critical organs or organs at risk, at acceptable levels.

In prior art techniques for spatially fractionated radiotherapy, this dose-based optimisation process is performed separately to the process of dividing the target volume into the sub-targets. In the prior art, the dose-based optimisation does not have any effect on how the target volume is divided into sub-targets.

Rather, in the prior art, the location, size, shape and number of the sub-targets is determined by trial-and-error, or by oncology professionals using their clinical experience. Radiotherapy treatment planners may, for example, constrain the location of the sub-targets by defining a desired distance (or "margin") of each sub-target to a boundary of the overall target volume. Similarly, another variable that may be constrained is the distance (or "margin") of each sub-target to neighbouring spherical targets. A geometrical relaxation algorithm may be used to iteratively adjust centres of the sub-targets to provide more effective distancing between separate sub-targets. However, all these methods use trial and error, clinical experience or heuristic methods to define the location, size, shape and number of the sub-targets. Further, the sub-targets characteristics are determined independently of the dose objective optimisation.

Thus, prior art methods do not take into account how the division of a target volume into sub-targets affects the dose distribution provided by the radiotherapy treatment plan. Conversely, prior art methods do not take into account how optimising the dose distribution affects the optimal division of a target volume into sub-targets. This is because the dose-based optimisation (using, e.g. cost functions), is performed separately to the division of the target volume into sub-targets. In other words, the process of dose-based optimisation of the treatment plan does not affect the process of dividing the target into sub-targets, and the process of dividing the target into sub-targets does not affect the process of dose-based optimisation of the treatment plan.

For example, in the prior art, the division of the target into sub-targets does not take into account how said division adversely affects the dose delivered to an organ at risk or a critical organ. A first manner of dividing the target into sub-targets may result in a dose distribution that is more favourable for an organ at risk, than a second manner of dividing the target into sub-targets would be for the organ at risk. Also, different manual, trial and error or heuristical ways of dividing the target into sub-targets may lead to different levels of dose-bridging (i.e., irradiation of healthy tissue located between targets).

Thus, in the prior art, there is no way of optimising a treatment plan that takes into account how dose levels can be affected by different ways of dividing the target volume into sub-targets. There is thus a need to provide improved treatment planning methods for spatially fractionated radiotherapy.

### SUMMARY OF THE INVENTION

In accordance with a first aspect of the invention, there is provided a method for generating and optimising a radiotherapy treatment plan for spatially fractionated radiotherapy, the method as defined by claim 1.

In accordance with a second aspect of the invention, there is provided a radiation treatment planning computer system as defined by claim 14.

In accordance with a third aspect of the invention there is provided a computer program product as defined by claim 15.

Optional features are defined by the dependent claims.

In accordance with the first aspect of the invention, there is provided a method for generating and optimising a radiotherapy treatment plan for spatially fractionated radiotherapy, the method comprising: constructing a model of patient anatomy, the model comprising a target volume and a plurality of sub-targets, wherein the sub-targets are determined by dividing the target volume; and generating the treatment plan by simultaneously optimising a combination of both (i) dose objectives and (ii) sub-targets characteristics.

Traditionally, dose objectives are defined and optimised separately to the definition of the sub-targets characteristics. By optimising the combination of both dose objectives and sub-targets characteristics simultaneously, optimising for dose objectives may impact the sub-targets characteristics, and optimising the sub-targets characteristics may impact the optimising of the dose objectives. In this way, an overall optimal solution is obtained that optimises the overall combination of dose objectives and sub-targets characteristics.

In this way, the choice of sub-targets characteristics is made to optimally affect the dose distribution of the treatment plan. For example, the sub-target characteristics may be optimised to minimise the dose to organs at risk. In prior art methods, the optimising of sub-targets characteristics does not take into account dose-based goals.

### Sub-targets characteristics

Optionally, the sub-targets characteristics include any combination of one or more of:
(i) location of each of the sub-targets;
(ii) shape of each of the sub-targets;
(iii) size of each of the sub-targets; and
(iv) number of the sub-targets.

This list of sub-targets characteristics is exemplary, and not exhaustive. Any number of sub-targets characteristics of any nature may be optimised. In some cases, all the above listed sub-targets characteristics are optimised simultaneously and in combination with the dose-based objectives. In other cases, only one of the sub-targets characteristics is optimised simultaneously and in combination with the dose-based objectives. In still other cases, any combination of two or three of the above listed sub-targets characteristics may be optimised simultaneously and in combination with the dose-based objectives.

Advantageously, whereas in the prior art the location, shape, size and number of the sub-targets was chosen manually, by trial and error, by clinical experience, by heuristic methods and separately from the dose optimisation, embodiments of the present invention can optimise location, shape, size and number of the sub-targets automatically by means of an optimisation process that includes dose-based optimisation.

### Dose objectives

Optionally, the dose objectives comprise at least minimum dose to the target volume and/or maximum dose to an organ at risk.

The dose objectives may be associated with clinical goals which may specify that in the eventual treatment plan, a particular plan metric should be greater or smaller than a given threshold value. Some examples of clinical goals include Volume-at-dose, Dose-at-Volume, Max Dose, Min Dose, Mean dose, Geometrically equivalent uniform dose (GEUD), Max dose at distance, Conformality, Homogeneity. These are non-limiting examples and the skilled person will be aware of any number of types of clinical goals and dose objectives that may be optimised in the generation of a radiation treatment plan. Dose objectives are discussed in more detail below.

Optionally, the simultaneously optimising a combination of both dose objectives and sub-target characteristics, further comprises simultaneously optimising a combination of dose objectives, sub-target characteristics and any combination of:
fluence;
control points; and
multi-leaf collimator sequences;
   of the treatment plan.

Advantageously, by simultaneously optimising the combination of sub-target characteristics and factors such as dose, fluence, control points and multi-leaf collimator sequences, the optimisation of each such factor may influence the optimisation of the sub-target characteristics. This is in contrast with prior art schemes where factors such as dose, fluence, control points and multi-leaf collimator sequences are generally optimised separately and independently from the optimisation of sub-target characteristics.

### Objective functions

The present discussion refers to minimising or maximising the value of an objective function. The objective function may be a cost function, or alternatively, a utility function. A cost function comprises cost terms and optimisation involves minimising the cost function. A utility function comprises reward terms and optimisation involves maximising the utility function. While certain parts of the discussion below refer to cost functions or cost terms, it should be understood that these can be replaced with utility functions or reward terms instead.

Optionally, the optimising comprises minimising or maximising the value of an objective function, wherein the objective function comprises at least one term related to the dose objectives, and at least one term related to the sub-targets characteristics. For example, the optimising may comprise minimising the value of a cost function, wherein the cost function comprises at least one cost term related to the dose objectives, and at least one cost term related to the sub-targets characteristics.

Optionally, the objective function comprises at least one term related to minimum dose for a target and/or maximum dose to an organ at risk. For example, the cost function may comprise at least one cost term related to minimum dose for a target and/or maximum dose to an organ at risk.

So, the present disclosure is concerned with objective functions of two types of cost or reward terms: dose-based terms and sub-targets characteristics terms. Hitherto, these terms have not been used in a single objective function. Advantageously, by means of the present disclosure, by including both types of terms in a single objective function, the optimisation of the first type of terms may affect the optimisation of the second type of term, to provide an overall optimised and minimised cost, or maximised reward. This was not possible in the prior art wherein dose objectives and sub-targets characteristics were chosen or optimised in separate and unrelated processes.

Optionally, the objective function comprises at least one term related to the location of any of the sub-targets. For example, the cost function may comprise at least one cost term related to the location of any of the sub-targets.

Optionally, the location of each sub-target comprises coordinates of a centre of the respective sub-target.

Advantageously, the location of the sub-targets may be optimised in the same objective function as the dose-based objectives.

Optionally, the objective function includes at least one term related to a distance between a sub-target and a boundary of the target volume. For example, the cost function may include at least one cost term related to a distance between a sub-target and a boundary of the target volume.

Advantageously, the distance between sub-targets and the boundary of the target volume may be optimised in the same objective function as the dose-based objectives.

Optionally, the objective function includes at least one term related to an inter-target distance between two sub-targets in a plane normal to the beam axis. For example, the cost function may include at least one cost term related to an inter-target distance between two sub-targets in a plane normal to the beam axis.

Throughout this disclosure, the phrase "inter-target distance" is used to refer to the distance between two sub-targets (not the distance between two overall target volumes).

The prescription dose distribution for the sub-targets may be asymmetrical in different directions, for example, in directions x, y or z in a three-dimensional coordinate system. Thus, when a clinician manually divides the target volume into sub-targets, the distance of each sub-target to its neighbouring targets may be different in the three directions of the coordinate system.

Advantageously, the inter-target distance between two sub-targets in a plane normal to the beam axis may be optimised in the same objective function as the dose-based objectives.

Optionally, the objective function includes at least one term related to an inter-target distance between two sub-targets in a direction parallel to a beam axis. For example, the cost function may include at least one cost term related to an inter-target distance between two sub-targets in a direction parallel to a beam axis.

Advantageously, the inter-target distance between two sub-targets in a direction parallel to the beam axis may be optimised in the same objective function as the dose-based objectives.

### Dose-fit cost terms

Optionally, the objective function includes at least one term related to how the sub-targets fit into a dose distribution comprising a prescription dose to targets and organs at risk. For example, the cost function may include at least one cost term related to how the sub-targets fit into a dose distribution comprising a prescription dose to targets and organs at risk.

That is, when the location, size or shape of the sub-targets is such that the surface of the sub-targets coincides with the dose distribution, the sub-targets advantageously fit well with the dose distribution of the plan. A poor fit would be associated with a high cost and an accurate fit would be associated with a low cost.

Advantageously, by fitting the sub-targets as much as possible to the dose distribution, the pressure felt, during planning, at the boundaries of the sub-targets to reduce healthy tissue dose and increase target dose is more uniform, allowing for a treatment plan that can conform to both the dose distribution and the sub-target shape.

Optionally, the objective function includes at least one term related to the extent to which a prescription dose isodose surface conforms to a surface of a sub-target. For example, the cost function may include at least one cost term related to the extent to which a prescription dose isodose surface conforms to a surface of a sub-target.

The prescription dose isodose surface relates to the dose that a clinician requires is delivered to the target volume. An isodose surface relates to a contour in three-dimensional space at which a dose is constant. Thus, the prescription dose isodose surface relates to a contour in three-dimensional space at which the prescription dose is constant.

Advantageously, by including this term in the objective function, the plan optimisation process is guided towards providing sub-targets locations, size and shape characteristics that conform to the prescription dose isodose surface, which in turn allows the generation of a treatment plan that can conform to both the prescription dose isodose surface and the sub-target surface.

Optionally, the objective function includes at least one term that is an integral of square distances between points on the prescription dose isodose surface and points on a surface of a sub-target. For example, the cost function may include at least one term that is an integral of square distances between points on the prescription dose isodose surface and points on a surface of a sub-target.

Advantageously, the integral of square distances between points on a prescription dose isodose surface and points on a surface of the sub-target reflects the extent to which the sub-target surface, as defined by sub-target location, shape and size, conforms to the contour formed by the prescription isodose surface.

This method of quantifying the conformity of the sub-target surface to the prescription dose isodose surface is exemplary and any mathematical method that characterises the deviation of the sub-target surfaces from the prescription dose isodose surface may be used.

Optionally, the objective function includes at least one term that relates to deviation of (i) a distance between a sub-target centre and a prescription dose isodose surface, from (ii) an average distance between the sub-target centre and the prescription dose isodose surface. For example, the cost function may include at least one cost term that penalises deviation of (i) a distance between a sub-target centre and a prescription dose isodose surface, from (ii) an average distance between the sub-target centre and the prescription dose isodose surface.

Here, it is noted that the average distance between the sub-target centre and the prescription dose isodose surface is a constant. Thus, this average distance may be represented as a circle around the sub-target, with the centre of the circle coinciding with the centre of the sub-target. The deviation of a distance between a sub-target centre and a prescription dose isodose surface from this average distance may then be viewed as deviations of this distance from the circle.

In some cases, the deviation of the distance of the sub-target surface to the circle representative of the average distance may be integrated (in case of a continuous distance) or summed (in case of discrete distances) to obtain the value of the cost or reward term that penalises the deviation from, or rewards adherence to, the average distance.

### Optimisation - further aspects

Optionally, said optimising includes adding and/or removing sub-targets.

Adding sub-targets to the model of the target volume would require smaller sub-targets that are more densely distributed, in order to fit into the overall target volume. Advantageously, this allows for more precise planning of the dose delivered to the sub-targets. Removing sub-targets from the model of the target volume would require larger sub-targets that are more sparsely distributed, in order to fill up the overall target volume. Advantageously, this allows for treatment planning that requires less computational resources and that requires less radiation treatment system resources. This trade-off between using more or fewer sub-targets may also be optimised as part of the characteristics of the sub-targets.

Optionally, the optimising comprises one or more of:
(i) gradient based optimisation;
(ii) simulated annealing;
(iii) differential evolution; and
(iv) column generation.

A skilled person will be aware of how to apply the above optimising algorithms to the objective functions, cost functions or utility functions of the present disclosure, and so these optimising algorithms are not discussed in detail here. Further, this list of possible optimisation algorithms is exemplary and not limiting. The objective function may be optimised by means of any suitable algorithm.

Optionally, the shape of one or more of the sub-targets is one or more of:
(i) spherical;
(ii) cylindrical; and
(iii) ellipsoid.

Spherical sub-targets are defined by their radius and the optimisation of the size and/or shape of such sub-targets proceeds by optimising the value of the radii of the spherical sub-targets.

Ellipsoidal sub-targets are achieved by using different cost or reward terms for sub-target radii in different directions (e.g. the x, y or z directions). This results in controllable and optimisable aspect ratios of the ellipsoidal shapes. Further, ellipsoidal shapes of different orientations may be achieved by changing the definition of the x, y or z directions, for example by rotating the x, y or z coordinate system.

Cylindrical sub-targets are achieved by modifying the cost or reward term to exclude from the inter-target distance cost or reward term any dependence on a particular (e.g. z) direction. Further, the length of the cylindrically shaped sub-targets may be optimised by introducing cost or reward terms that include the start and end position of a cylinder in a particular direction. Use of the cost terms or reward terms associated with start/end positions of a cylinder provides a sub-target with optimal cylindrical geometry.

Some examples of sub-target shape have been discussed above. In principle, any shape of sub-target may be used, although some shapes are more advantageous than others. Generally, symmetrical shapes, and especially "roundish" symmetrical shapes are preferable as this avoids directional asymmetry in the optimisation.

### Representing the sub-target

Optionally, the location of at least one of the sub-targets comprises a volume mask for the at least one sub-target, and the method further comprises updating the masks during optimisation.

That is, the model of the overall target volume may comprise a three-dimensional image made up of voxels. A volume mask may then be used to label each voxel as belonging to a particular sub-target. This location information can then be used in any of the sub-target location optimisation steps described herein.

Optionally, the location of at least one of the sub-targets is defined by means of a single distance function wherein a value of each lattice point of the single distance function comprises a shortest distance to surfaces of the sub-targets.

The use of a single distance function is an alternative way of defining the location of the sub-targets, and is computationally less expensive than using a volume mask to define the location of the sub-targets.

Optionally, the treatment plan is for volumetric modulated arc therapy, VMAT.

However, the present radiotherapy treatment planning method may be applied in any type of radiotherapy, including intensity modulated radiotherapy (IMRT).

### Other aspects of the invention

In accordance with the second aspect of the invention, there is provided a radiation treatment planning computer system comprising a memory and a processor configured to perform the method as defined above.

In accordance with the third aspect of the invention, there is provided a computer program product comprising a non-transitory, computer readable storage medium encoded with instructions operable for execution by a processor to perform the method as defined above.

In accordance with a fourth aspect of the invention, there is provided means for generating and optimising a radiotherapy treatment plan for spatially fractionated radiotherapy, the means comprising:
means to construct a model of patient anatomy, the model comprising a target volume and a plurality of sub-targets, wherein the sub-targets are determined by dividing the target volume;
   and
means for generating the treatment plan by simultaneously optimising a combination of both (i) dose objectives and (ii) sub-targets characteristics.

An example of the means for generating and optimising the radiotherapy treatment plan, and the means for constructing the model, is the computer system described below.

### BRIEF DESCRIPTION OF THE DRAWINGS

For a better understanding of the present disclosure, embodiments will now be described by way of example with reference to the accompanying drawings.
Figure 1 shows a block diagram of an example of a computing system upon which some embodiments described herein may be implemented.
Figure 2 is a block diagram showing selected components of a radiation treatment system upon which some embodiments according to the present invention can be implemented.
Figure 3 illustrates elements of an exemplary radiation treatment system upon which radiation treatment plans generated according to some embodiments of the present invention may be implemented.
Figure 4A illustrates the division of an overall target volume into sub-targets in accordance with spatially fractionated radiotherapy.
Figure 4B illustrates two sub-targets of an overall target volume.
Figure 5A illustrates a cost term relating to a distance of a sub-target to a boundary of the overall target volume.
Figure 5B illustrates a cost term relating to a distance of a sub-target to other sub-targets.
Figure 5C illustrates isocosts associated with inter-target distance in the z direction and inter-target distance in the xy plane.
Figure 5D illustrates a cost term relating to a distance of a sub-target to a boundary of the overall target volume, as affected by sub-target size.
Figure 5E illustrates a cost term relating to a distance of a sub-target to other sub-targets, as affected by sub-target size.
Figure 6 is a flowchart illustrating steps within each iteration of the optimisation process.
Figure 7 is a flowchart illustrating the steps of constructing an objective function having both dose-based cost terms and sub-targets characteristics cost terms, and the subsequent optimisation of the constructed objective function.

### DETAILED DESCRIPTION

Reference will now be made in detail to several embodiments. While the subject matter will be described in conjunction with the specific embodiments, it will be understood that they are not intended to limit the claimed subject matter to these embodiments. On the contrary, the claimed subject matter is intended to cover alternatives, modifications, and equivalents, which may be included within the scope of the claimed subject matter as defined by the appended claims.

Furthermore, in the following detailed description, numerous specific details are set forth in order to provide a thorough understanding of the claimed subject matter. However, it will be recognised by one skilled in the art that embodiments may be practised without these specific details or with equivalents thereof. In other instances, well-known methods, procedures, components, and circuits have not been described in detail as not to unnecessarily obscure aspects and features of the subject matter.

The following description is presented to enable a person skilled in the art to make and use the embodiments of this invention; it is presented in the context of a particular application and its requirements. Various modifications to the disclosed embodiments will be readily apparent to those skilled in the art, and the general principles defined herein may be applied to other embodiments and applications without departing from the scope of the present disclosure. Thus, the present invention is not limited to the embodiments shown, but is to be accorded the widest scope consistent with the principles and features disclosed herein.

### Computer system

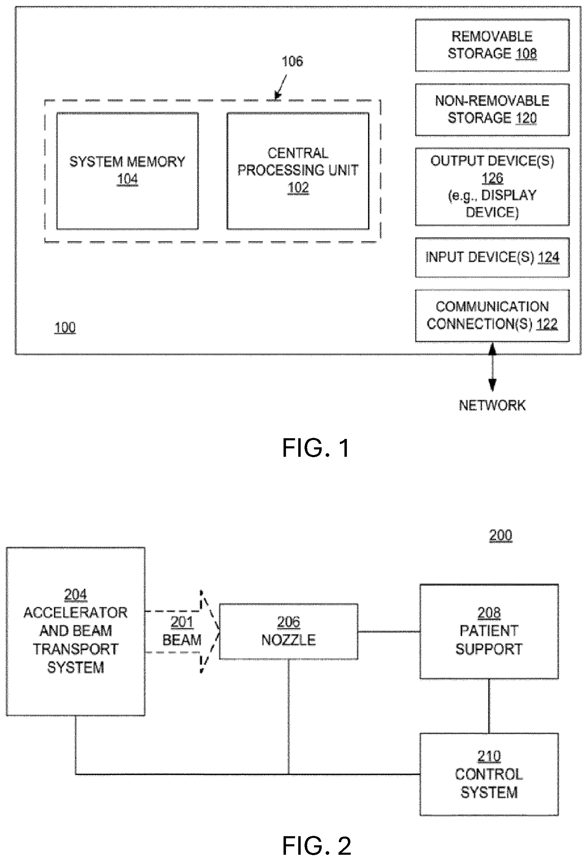

Figure 1 shows a block diagram of an example of a computing system 100 upon which the embodiments described herein may be implemented. In a basic configuration, the system 100 includes at least one processing unit 102 and memory 104. This most basic configuration is illustrated in Figure 1 by dashed line 106. The system 100 may also have additional optional features and/or functionality. For example, the system 100 may also include additional storage (removable and/or non-removable) including, but not limited to, solid state, magnetic or optical disks or tape. Such additional storage is illustrated in Figure 1 by removable storage 108 and non-removable storage 120. The system 100 may also contain communications connection(s) 122 that allow the device to communicate with other devices, e.g., in a networked environment using logical connections to one or more remote computers.

The system 100 also includes input device(s) 124 such as keyboard, mouse, pen, voice input device, touch input device, etc. Output device(s) 126 such as a display device, speakers, printer, etc., are also included.

In the example of Figure 1, the memory 104 includes computer-readable instructions, data structures, program modules, and the like. Depending on how it is to be used, the system 100 - by executing the appropriate instructions or the like - can be used to implement a method for generating and optimising a radiotherapy treatment plan for spatially fractionated radiotherapy, as disclosed herein.

### Radiation treatment system

Figure 2 is a block diagram showing selected components of a radiation treatment system 200 upon which embodiments according to the present invention can be implemented. In the example of Figure 2, the system 200 includes an accelerator and beam transport system 204 that is operable to generate and/or accelerate a beam 201. The accelerator and beam transport system can generate and deliver beams of various types including, for instance, X-ray (photon) beams, proton beams, electron beams, ion beams, or atomic nuclei beams (e.g., using elements such as carbon, helium, or lithium). The operations and parameters of the accelerator and beam transport system 204 are controlled so that the intensity, energy, size, and/or shape of the beam are dynamically modulated or controlled during treatment of a patient according to an optimised radiation treatment plan produced by and stored within system 100 as discussed above.

The nozzle 206 is used to aim the beam toward various locations (e.g., of a target) within a patient supported on the patient support device 208 (e.g., a chair, couch, or table) in a treatment room. A target may be an organ, a portion of an organ (e.g., a volume or region within the organ), a tumour, diseased tissue, or a patient outline, for instance.

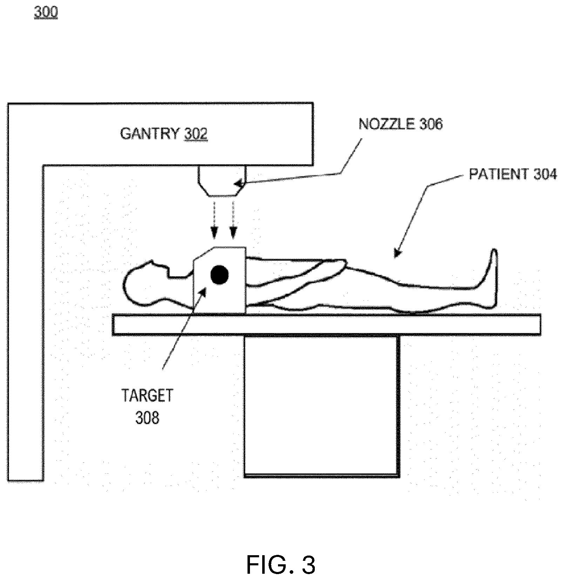

The nozzle 206 may be mounted on or may be a part of a gantry structure (Figure 3) that can be moved relative to the patient support device 208, which may also be moveable. The accelerator and beam transport system 204 may be mounted on or are a part of the gantry structure; alternatively, the accelerator and beam transport system are separate from (but in communication with) the gantry structure.

The control system 210 of Figure 2 receives and implements a prescribed treatment plan which is generated and/or optimised according to embodiments of the present invention. The control system 210 includes a computing system having a processor, memory, an input device (e.g., a keyboard), and optionally a display; the system 100 of Figure 1 is an example of such a platform for the control system 210. The control system 210 can receive data regarding the operation of the system 200. The control system 210 can control parameters of the accelerator and beam transport system 204, nozzle 206, and patient support device 208, including parameters such as the energy, intensity, size, and/or shape of the beam, direction of the nozzle, and position of the patient support device (and the patient) relative to the nozzle, according to data the control system 210 receives and according to the radiation treatment plan.

Figure 3 illustrates exemplary elements of a radiation treatment system 300 for treating a patient 304. The system 300 is an example of an implementation of the radiation treatment system 200 of Figure 2. The gantry 302 may rotate around an axis, e.g. the gantry may rotate around a patient on a couch. The patient may be moved by moving the couch. While the patient 304 is supine in the example of Figure 3, the invention is not so limited. For example, the patient 304 can instead be seated in a chair or positioned in any orientation. The gantry 302 can be controlled by a treatment system using an optimised treatment plan generated according to embodiments of the present invention.

### Prior art dose based objectives

As noted above, the present invention relates to simultaneously optimising a combination of both (i) dose objectives and (ii) sub-targets characteristics. Dose objectives in general are known in the prior art.

In particular, prior art dose-based objectives may relate to clinical goals that may specify that in the eventual treatment plan, a particular plan metric should be greater or smaller than a given threshold value. Some examples of clinical goals upon which the dose-based objectives may be based include Volume-at-dose, Dose-at-volume, Max dose, Min dose, Mean dose, Geometrically equivalent uniform dose (GEUD), Max dose at distance, Conformality and Homogeneity. A skilled person will be aware of the definition of such clinical goals and so these are not described in detail herein.

### Spatially fractionated radiotherapy

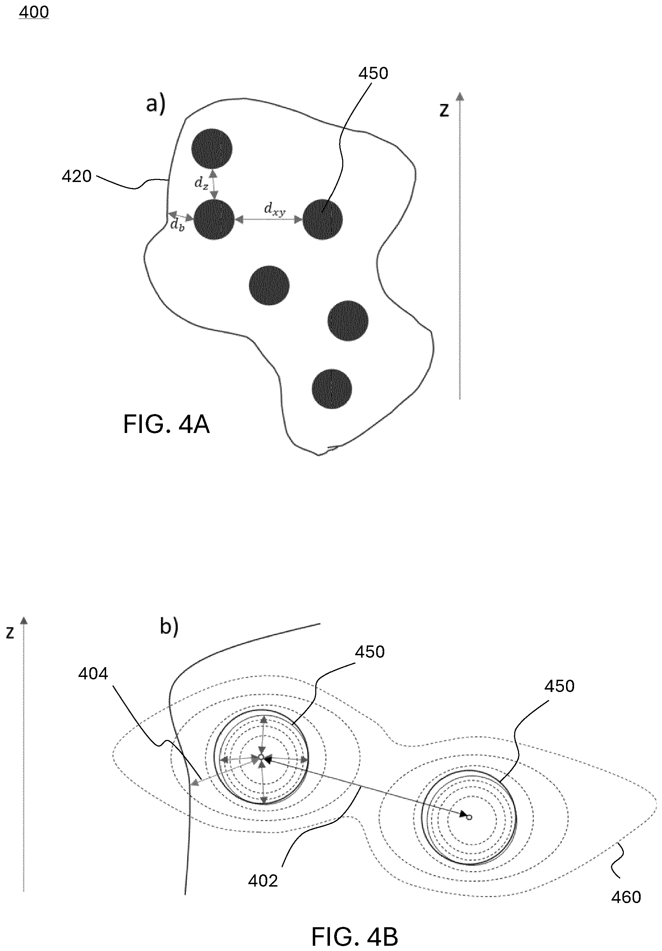

Figure 4A illustrates an example of a target volume 400 divided into a plurality of sub-targets 450. That is, in Figure 4A, the curve 420 represents the contour of the overall target volume 400, while the solid circles represent the sub-targets 450. The manner in which the division is made affects the characteristics of the sub-targets, such as the location, shape, size or number of the sub-targets. Conventionally, the division of the overall target volume into sub-targets is done using trial and error, experimental knowledge, clinical experience or heuristic methods. The present disclosure allows the division of the overall target volume into sub-targets to take place by means of optimisation of a cost function that includes dose-based objectives.

In an example, the location of each sub-target may be constrained by a defined distance from the sub-targets to a boundary of the overall target volume, which is denoted "d_{b}" in Figure 4A. This distance may be defined as the distance between the centre of a sub-target to a boundary of the overall target volume.

In another example, the location of each sub-target may be constrained by a defined distance from the sub-targets to respective neighbouring sub-targets, also referred to as "inter-target" distance, and denoted "dᵢₜ". As the desired dose distribution is generally asymmetric in different directions, three different inter-target distances may be defined in each direction of a three-dimensional 3D coordinate system, x, y, z. These inter-target distances may be referred to as dₓ, d_{y} and d_{z}. In some cases, there is asymmetry in the dose distribution in a first direction and the dose distribution in a plane orthogonal to the first direction. Here, the inter-target distance may be defined as a distance between sub-targets in a plane, such as an xy-plane. In this example, the inter-target distance may be referred to as d_{xy}.

Figure 4A shows examples of inter-target distances d_{z} and d_{xy}. In the example of Figure 4A, the x, y and z directions refer to the following:
x - out of the paper towards the eye
y - towards the right
z - towards the top

It can be seen in Figure 4A that the direction d_{xy} is illustrated as towards the right. This is because the length in the x direction is coming out of the paper towards the eye but the length in the y direction is towards the right; as the length in the x direction is not visible on paper, this distance viewed from above only shows the length in the y direction and so the distance appears as a line going towards the right.

Figure 4B illustrates two sub-targets 450. The distance between the sub-targets 450 is shown by the line 402 and may be denoted as dᵢₜ. The distance from the sub-target 450 to the boundary of the overall target volume is shown by the line 404 and may be denoted d_{b}.

In the examples of Figure 4A and Figure 4B, the only variables with regard to the sub-targets are the distance to the boundary of the target volume, and the inter-target distance. These variables may be used to describe the location of the sub-targets. However, in dividing the overall target volume into sub-targets, variables other than sub-target location may be of interest: that is, the shape, size and number of the sub-targets are also important (these are not shown in the drawings).

### Optimising dose and sub-targets simultaneously

As explained above, in the prior art, the dose-based optimisation is a separate and distinct process to the process of dividing the overall target volume into sub-targets. In contrast, according to the present disclosure, the optimisation of the treatment plan is performed by simultaneously optimising a combination of both (i) dose objectives and (ii) sub-targets characteristics. In this way, the optimisation of the dose affects the optimisation of the sub-targets characteristics, and the optimisation of the sub-targets characteristics affects the optimisation of the dose. The result of the overall optimisation process is a solution that provides a particular division of the target volume into sub-targets that is optimal for providing the optimal dose distribution.

In other words, in the present disclosure, during dose-based optimisation, the characteristics of the sub-targets is also optimised together with dose. So, during iterative optimisation, the sub-targets characteristics, such as location or shape, are made to change along with the other parameters that are being optimised. These parameters may include machine parameters, which may be, for example, multileaf collimator leaf sequences, start/stop gantry angle range, collimator angle or couch position. The dose that is delivered according to the treatment plan would depend on these machine parameters. That is, in attempting to optimise dose, the various machine parameters that deliver the dose, such as multileaf collimator leaf sequences, are optimised so as to achieve a dose that is as close as possible to the dosimetrical objectives. These dose objectives may comprise, for example, a maximum dose for an organ at risk, or a minimum dose for a target.

By optimising dose in the same optimisation process as optimising the sub-targets characteristics, the optimiser may seek sub-targets characteristics that result in favourable adherence to dose-based goals, such as avoiding OARs or dose-bridging between targets (i.e. the reduction of dose to healthy tissue located between targets). Further, the optimiser may seek sub-targets characteristics that reduce dose-bridging between the sub-targets themselves. Dose-bridging between sub-targets is illustrated in Figure 4B which shows dose being delivered to the regions between the sub-targets.

In addition to optimising sub-targets characteristics simultaneously with optimising dose objectives, in some embodiments this simultaneous optimisation may be combined with determining aspects of the sub-targets characteristics using clinical or heuristic experience.

### Cost function based SFRT optimisation - location of sub-targets

The simultaneous optimisation of dose and sub-targets characteristics within the same optimisation process may be performed using a cost function. In particular, this simultaneous optimisation may comprise minimising the value of a cost function, wherein the cost function comprises at least one cost term related to dose objectives, and at least one cost term related to the sub-targets characteristics.

In particular, the cost function may include cost terms related to dose objectives such as minimum dose to a target volume or maximum dose to an organ at risk, and the same cost function may include cost terms related to sub-targets characteristics such as sub-target location, shape, size or number.

An exemplary cost function may be: $C ′ = C + {\sum }_{k} {c}^{b} \left({d}_{b , k}\right) + {\sum }_{k , l} {c}^{it} \left({d}_{z , kl}, {d}_{xy , kl}\right) + {\sum }_{k} {c}^{dose - fit}$ Here:
C' = the overall, combined cost function
C = prior art cost function
c^{b} = boundary-based cost term
d_{b,k} = distance of sub-target k to boundary of overall target volume
c^{it} = inter-target based cost term
d_{z,kl} = distance between sub-target k and sub-target l in the z direction
d_{xy,kl} = distance between sub-target k and sub-target l in the xy plane
c^{dose-fit} = extent to which sub-targets fit into the dose distribution

The overall cost function C' is a sum of the prior art cost function C and the cost terms according to the present disclosure. The prior art cost function C may include standard cost terms such as those related to deviation from dosimetrical goals and control-point related contributions to cost such as costs associated with limits for the maximum monitor unit levels or costs associated with the multileaf collimator aperture shapes. The cost terms according to the present disclosure in the overall cost function C' include (i) a boundary distance based cost term c^{b}, (ii) an inter-target distance cost term c^{it} and (iii) a cost term c^{dose-fit} reflecting the extent to which the sub-targets fit into the dose distribution. The distance between the boundary of the target volume and a sub-target k is denoted d_{b,k}; the distance between target k and target l in the z direction is denoted d_{z,kl}; and the distance between target k and target l in the xy plane is denoted d_{xy,kl}.

The purpose of the boundary distance cost term c^{b} and the inter-target distance c^{it} is to maintain a desired distance between the sub-targets and the boundary of the overall target volume, or to maintain a desired distance between different sub-targets.

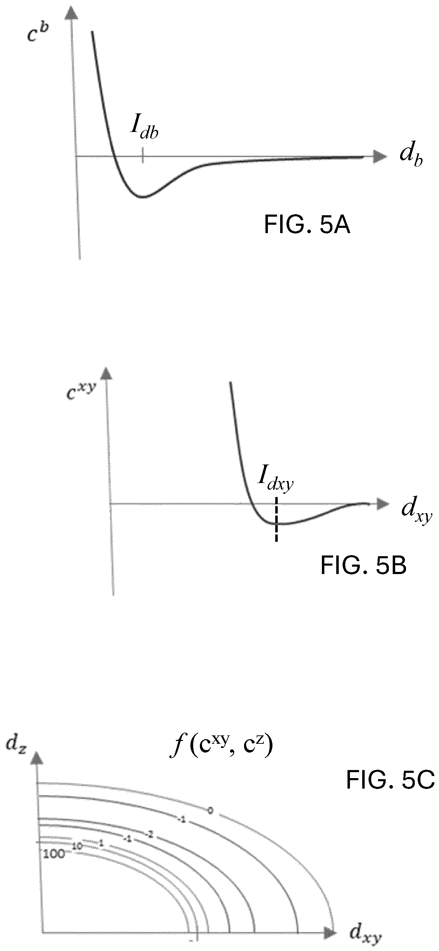

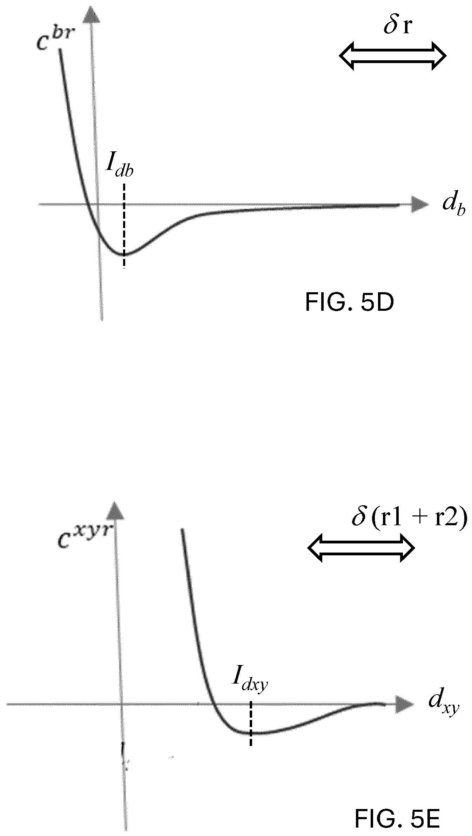

Figure 5A illustrates the cost function c^{b}, Figure 5B illustrates the cost function c^{xy} and Figure 5C illustrates a combination of the cost functions c^{xy} and c^{z}. In general, these cost functions and the other cost functions described e.g. in relation to Figures 5D and 5E, may be constructed by considering the relative cost of different ranges of each corresponding sub-target characteristic. For example, sub-target sizes and shapes that result in the sub-target extending out of the overall target volume are assigned a very high cost as this is not desirable; sub-target locations very far away from the overall target volume boundary will be assigned a low cost as inner sub-targets generally do not affect the boundary cost. The shape of the various cost functions may be determined, manually or automatically, using such variables and factors.

Figure 5A illustrates the cost c^{b} varying with the distance d_{b} between a sub-target and the overall target boundary. The distance d_{b} is measured as the distance from the centre of a sub-target to the boundary of the overall target. It can be seen that the cost c_{b} has a global minimum value, and as this is the lowest cost, it is "ideal", and labelled l_{db}. For example, it may be desired that the boundary of the sub-target makes contact with the boundary of the overall target volume. Thus, in this example, the ideal distance from the centre of the sub-targets to the boundary of the overall target volume would be equal to the radius of the (spherical) sub-targets, or r. That is, if the distance between the centre of the sub-targets and the boundary of the overall target volume is equal to the radius r of the spherical sub-targets, then the boundary of the sub-targets would make contact with the boundary of the overall target volume. This is advantageous because it reduces or minimises the amount of the overall target volume that is not covered by the sub-targets.

When the distance between the centre of the sub-targets and the boundary of the overall target value is less than the radius of the sub-targets, then the value of d_{b} is lower than r and this means that a part of the sub-target would actually be outside the target volume. As it is not desirable to irradiate tissue outside the target volume, the cost associated with values of d_{b} less than r is very high.

Targets towards the inner part of the target volume are more distant from the boundary of the target volume than r, and thus have a higher value for d_{b}. Much larger boundary distances do not affect the cost associated with the sub-targets characteristics, so such large distances to the boundary have a cost that approaches zero. A cost of zero reflects that such large distances between the sub-targets and the boundary are unimportant. In contrast, the cost of an ideal distance r to the boundary is negative, which means such a distance is advantageous. As noted above, the cost of a distance to the boundary that is less than r is high, which means that such a distance is disadvantageous.

In some cases, the radiotherapy planning system may not include, in the overall cost function, a boundary cost term for sub-targets deep inside the target. This is because the boundary cost associated with such sub-targets is very low (as explained above) and so may be effectively ignored.

Figure 5B illustrates the cost c^{xy} associated with an inter-target distance d_{xy} in the xy plane. The distance d_{xy} is measured as the distance from the centre of a sub-target to the centre of a neighbouring sub-target (in the xy plane). Again, in the present example, the sub-targets comprise spheres of radius r. Thus, in order to avoid two neighbouring sub-targets from overlapping each other, the inter-target distance should be at least 2r. Further, it may not be desirable that the boundaries of different sub-targets contact each other. Thus, each sub-target may be a minimum distance, α, from each other. Thus, the overall ideal distance from the centre of a first sub-target and the centre of a second sub-target may be 2r + α.

Thus, in Figure 5B, it can be seen that the distance d_{xy} that is associated with the lowest cost is labelled as l_{dxy}. l_{dxy} may be equal to twice the radius of the (spherical) sub-targets plus a constant, that is, 2r + α. Values of d_{xy} lower than 2r + α are associated with higher cost. Values of d_{xy} lower than 2r imply that the two sub-targets actually overlap and are thus associated with very high costs. Values of inter-target distances higher than the exemplary ideal value of 2r + α have a higher (negative) cost but tend towards zero with increasing values of inter-target distances. This reflects that the planning system does not consider very large values of inter-target distances as important.

In some cases, the radiotherapy planning system may not include, in the overall cost function, an inter-target cost term for sub-targets that are very far apart, such as much further apart than 2r + α. This is because the inter-target costs associated with such sub-targets are very low (as explained above), and may effectively be ignored.

Figure 5C illustrates a graph of isocosts showing the isocosts associated with (i) inter-target distance in the xy plane, d_{xy}, and (ii) inter-target distance in the z plane, d_{z}. Seven isocost contours are shown, wherein each isocost contour reflects a particular cost value of a combination of d_{xy} and d_{z}.

It can be seen that for the lowest inter-target distances d_{xy} and d_{z} shown in Figure 5C, the isocost contour has a much higher cost - in this example, the cost is "100". This is because low inter-target distances d_{xy} and d_{z} mean that the sub-targets are overlapping, which is not desirable. For inter-target distances d_{xy} and d_{z} in a middle range of values, the isocost contour has negative cost - in this example, the costs of three middle range inter-target distances is -1, -2 and -1. This is because middle range inter-target distances d_{xy} and d_{z} mean that the sub-targets are near or about a distance of 2r + α apart, which is desirable. For inter-target distances d_{xy} and d_{z} higher or much higher than 2r + α, the isocost contour is "0", which reflects that such inter-target distances are relatively unimportant.

In some cases, the radiotherapy planning system may not include, in the overall cost function, inter-target cost terms c^{xy} or c^{z} for sub-targets that are very far apart, such as much further apart than 2r + α. This is because the inter-target cost associated with such sub-targets is very low (as explained above), and so may effectively be ignored.

### Cost function based SFRT optimisation - size and shape of sub-targets

The examples of Figures 5A-5C relate to optimising the location of the sub-targets, such as distance to boundary, or inter-target distance. In other embodiments, the size or shape of the sub-targets may also be optimised.

An exemplary cost function that includes optimisation of sub-target size may be: $C ′ = C + {\sum }_{k} {c}^{r} \left({r}_{k}\right) + {\sum }_{k} {c}^{br} \left({d}_{b , k}, {r}_{k}\right) + {\sum }_{k , l} {c}^{itr} \left({d}_{z , kl}, {d}_{xy , kl}, {r}_{k}, {r}_{l}\right) + {\sum }_{k} {c}^{dose - fit}$ Here:
C' = the overall, combined cost function
C = prior art cost function
c^{r} = cost term relating to sub-target size (in this example, radius r)
rₖ = radius of (e.g. spherical) sub-target k
rₗ = radius of (e.g. spherical) sub-target l
c^{br} = cost term of distance to boundary depending on sub-target radius
d_{b,k} = distance of sub-target k to boundary of overall target volume
d_{z,kl} = distance between sub-targets k and l in the z direction
d_{xy,kl} = distance between sub-targets k and l in the xy plane
c^{itr} = cost term of inter-target distance depending on sub-target radius
c^{dose-fit} = extent to which sub-targets fit into the dose distribution

The overall cost function C' is a sum of the prior art cost function C and the cost terms according to the present disclosure. The prior art cost function C may include standard cost terms such as those related to deviation from dosimetrical goals and control-point related contributions to cost such as costs associated with limits for the maximum monitor unit levels or costs associated with the multileaf collimator aperture shapes. The cost terms according to the present disclosure in the overall cost function C' of Equation 2 include (i) a sub-target size (e.g., sub-target radius) cost term c^{r}, (ii) a cost term c^{br} relating to the distance to boundary taking into account sub-target radius, (iii) a cost term c^{itr} relating to the inter-target distance taking into account sub-target radius and (iv) a cost term c^{dose-fit} reflecting the extent to which the sub-targets fit into the dose distribution. The radius of a sub-target k is denoted rₖ, the radius of a sub-target l is denoted rₗ, the distance between the boundary of the target volume and a sub-target k is denoted d_{b,k}; the distance between target k and target l in the z direction is denoted d_{z,kl}; and the distance between target k and target l in the xy plane is denoted d_{xy,kl}.

The cost term *c^{r}*(*rₖ*) relating to the sub-target size or radius may be written as: ${c}^{r} \left({r}_{k}\right) = {w}_{k} {\left({r}_{k}-\hat{{r}_{k}}\right)}^{2}$ Here:
$\hat{{r}_{k}}$ = ideal sub-target size
*wₖ* = a predefined weight coefficient

Thus, the cost term c^{r} is expressed in this example as penalising any deviation from the ideal sub-target size $\hat{{r}_{k}}$.

Figure 5D illustrates the cost c^{br} varying with distance d_{b} between a sub-target and the overall target boundary. The graph of Figure 5D is similar to the graph of Figure 5A, except that the graph shifts to the right or to the left in dependence on a change in the size of the sub-targets. If the size of a sub-target is represented by the radius r of the sub-target, and any change in the radius r is represented by δr, then the graph of Figure 5D would shift to the right or to the left in dependence on δr. If the ideal sub-target size is r, then the minimum cost would be associated with sub-target size r; then, if the sub-target size r changes by δr, the ideal sub-target size would also change by δr.

Figure 5E illustrates the cost c^{xyr} associated with inter-target distance d_{xy} as affected by sub-target size. The graph of Figure 5E is similar to the graph of Figure 5B, except that the graph shifts to the right or to the left in dependence on a change in the size of the sub-targets. If the size of a sub-target is represented by the radius r of the sub-target, and any change in the radius r is represented by δr, then the graph of Figure 5E would shift to the right or to the left in dependence on δr. If the ideal sub-target size is r, then the minimum cost would be associated with 2r+α, as discussed above in relation to Figure 5B; then, if the sub-target size r changes by δr, the ideal sub-target size would also change by δr.

In some embodiments, the optimisation of the sub-targets characteristics allows for different sub-targets having different sizes or radii. In other embodiments, all the sub-targets have the same size or radius.

Figures 5A-5E illustrate the cost terms associated with boundary distance, inter-target distance, the combination of boundary distance and inter-target distance, boundary distance as affected by the size of the sub-targets, and inter-target distance as affected by the size of the sub-targets. In some embodiments, in addition to such cost terms relating to sub-target location and size, further cost terms may be introduced in the overall cost function that may be used to optimise the shape of the sub-targets.

For example, by using different cost terms for sub-target radii in different directions (e.g. the x, y or z directions), ellipsoidal shapes may be achieved with controllable and optimisable aspect ratios. Further, the x, y or z directions may be rotated to achieve ellipsoidal shapes of different orientations.

As a further example, by modifying the cost terms to exclude from the inter-target distance cost term any dependence on a particular (e.g. z) direction, cylindrically shaped sub-targets may be achieved. Further, by introducing cost terms that include the start and end position of a cylinder in a particular direction, the length of the cylindrically shaped sub-targets can also be optimised.

In general, it is reasonable to use a relatively "roundish" shape for the sub-targets to avoid creating a disadvantageous directional asymmetry in the optimisation.

In addition to optimising the location, size and shape of the sub-targets, the number of sub-targets may also be optimised by including in the cost function a cost term related to the number of sub-targets. In particular, the minimum value or optimal value of the number of sub-targets may be specified, and the cost term would penalise any deviation from this minimum or optimal value.

### Cost function based SFRT optimisation - dosimetrical fit of sub-targets

In addition to optimising the location, size and shape of the sub-targets, it is advantageous to optimise how the sub-targets "fit" into the dose distribution of the radiation therapy treatment plan. This takes into account how the sub-target locations seem to fit in the general dose distribution which in turn is affected by the geometry of the organs at risk and the targets, and the need to avoid dose bridging. The extent to which the sub-targets "fit" into the dose distribution is represented by the cost term c^{dose-fit} in Equations 1 and 2 above.

More particularly, the asymmetry of the dose distribution in the vicinity of the surface of the sub-targets affects how the sub-target locations and shapes fit to the dose distribution produced by the standard cost function.

In some embodiments, a prescription-dose isodose surface is used to characterise the dose distribution close to the target surface. Ideally the prescription isodose surface would coincide with the (spherical) sub-target surface, or at least be symmetrically positioned with respect to the sub-target surface. The coinciding of the prescription isodose surface with the sub-target surface may be measured from the sub-target surface itself, or from an outer margin of the sub-target surface, such as a region that extends half a radius outside the sub-target surface, the radius referring to the (e.g. spherical) sub-target radius.

In some embodiments, the extent to which the sub-targets fit into the dose distribution is defined as the extent to which the minimum distances between the prescription dose isodose surface and all points on the surface of the sub-target, are equal.

The symmetrical coincidence of the sub-target surface with the prescription dose isodose surface would mean that during optimisation, the optimiser is pressured to reduce target dose to spare surrounding organs, in a uniform way. In other words, the cost c^{dose-fit} is calculated by observing the prescription dose iso-surface in the vicinity of the boundary of the spherical target, and by requiring the distance to be the same in all directions. If that is not the case, the plan optimiser will be directed to move the spherical target in a direction that would improve the fit to the dose distribution.

During optimisation of dose distribution there is a fundamental tension between reducing dose to the organs at risk and at the same time maintaining the dose to the target. Thus, during optimisation, the objectives to reduce the dose to the organs at risk compete with the objectives to maintain the target dose. If the prescription dose isodose surface is symmetric relative to the sub-target surface this 'pressure' is even and uniform at the sub-target surface, providing a better "fit" of the sub-target to the dose distribution.

In general, the c^{dose-fit} term is introduced in the cost function so that during optimisation the sub-target positions are optimised to fit as well as possible to the dose distribution.

In one embodiment, c^{dose-fit} is calculated as the square difference SD between the point in the prescription-dose isodose surface and the nearest point in the (spherical) sub-target surface. The total cost term may be obtained by integrating SD over all points in the prescription-dose isodose surface (in the vicinity of one particular spherical target). Also, a a relatively sparser sampling can be used, wherein the integration is performed over relatively more sparsely distributed points in the prescription-dose isodose surface.

In another embodiment c^{dose-fit} is calculated by first defining the average distance between the prescription-dose isodose surface and the centre-point of the spherical sub-target, and then any deviation from the distance and the average distance may be penalised.

In both implementations, it is important that if gradient-based optimisation methods are used, the cost term is implemented so that once derived relative to the spherical sub-target location, there is a gradient pressuring the optimiser to move the spherical target location to a more symmetric position.

### Optimiser loop

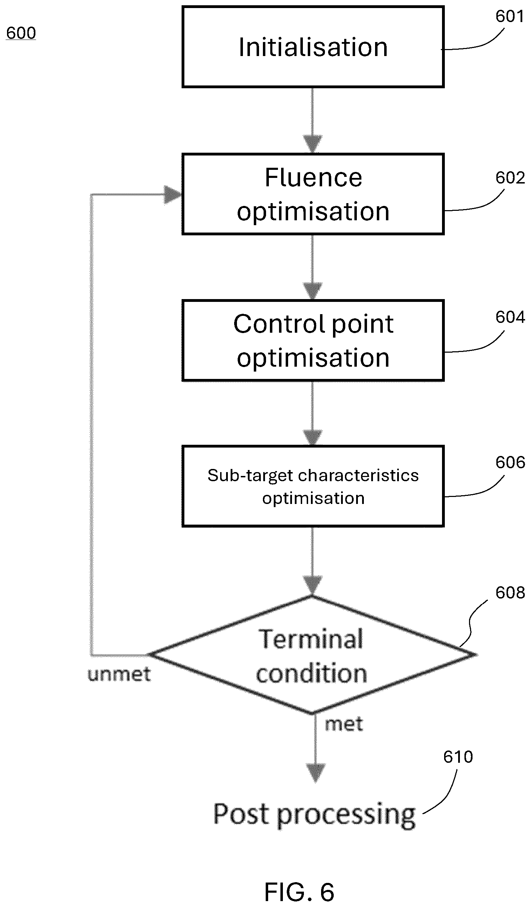

Figure 6 illustrates an exemplary flowchart indicating the iterative loop performed by the treatment plan optimiser in optimising a radiation therapy treatment plan. The flowchart shows that the iterative process begins after an initialisation step 601. Each iteration includes fluence optimisation 602 and control point optimisation 604, in accordance with the prior art. The iterative process continues until a terminal condition 608 is met.

According to embodiments of the present disclosure, the iterative loop further contains an additional step relating to sub-targets characteristics optimisation, 606. In this step, the characteristics of the sub-targets such as locations, shape, size and number are optimised or updated. Figure 6 shows that according to the present disclosure, the sub-targets characteristics optimisation step 606 is performed after the control point optimisation step 604 and before the terminal condition check step of 608. In variations of this method, the sub-targets characteristics optimisation step 606 may be performed at a different point in the iterative optimisation process, such as after the fluence optimisation step 602 and before the control point optimisation step 604.

In some embodiments, the fluence optimisation step 602 uses the updated sub-targets characteristics, as updated in step 606 of the previous iteration, to determine the cost associated with the dose distribution.

In some embodiments, the optimisation to improve the dosimetrical fit of the sub-targets to the dose distribution may be performed as part of the fluence optimisation step 602.

To optimise the treatment plan using a cost function as discussed above, the plan optimiser may use gradient based optimisation methods. Here, in the optimisation process, the sub-target locations, shapes and sizes are changed; if the cost falls, the sub-target locations, shapes and sizes continue to be changed in the same "direction"; if the cost rises, the change in sub-target locations, shapes and sizes changes direction. The shape of the cost function should be such that one could calculate the impact of very small (i.e. infinitesimally small) changes to the optimised parameters (such as sub-target locations, shapes and sizes). This would allow searching for better solutions in the close neighbourhood of the previous solution by responding in this way to small changes.

Alternatively, the plan optimiser may use non-gradient based optimisation methods suitable for treatment planning optimisation, such as simulated annealing, differential evolution, or column generation. The method described herein is not restricted to any particular optimisation method.

### Overall method

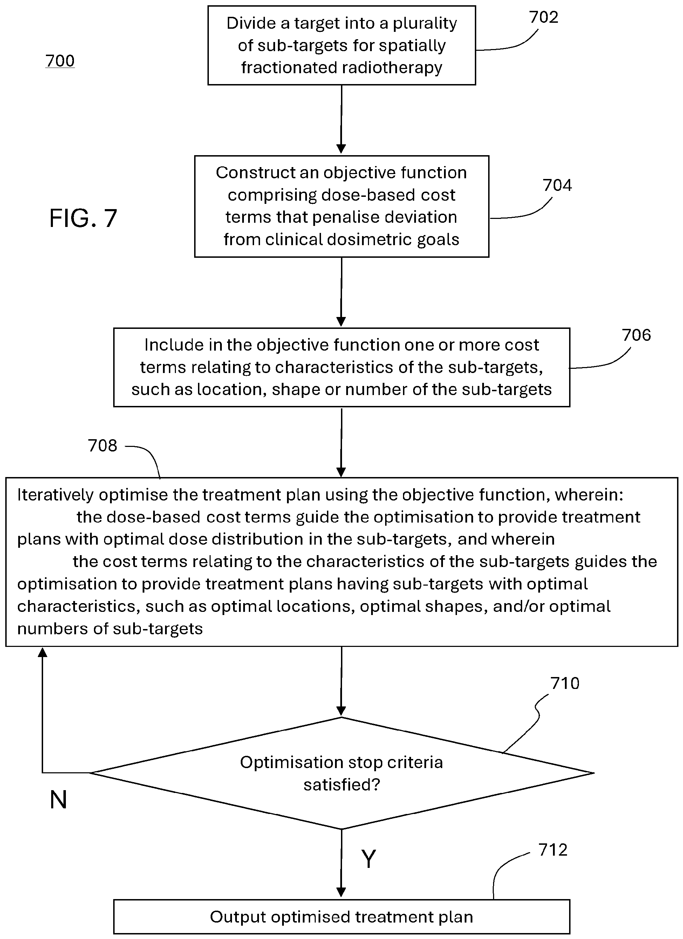

Figure 7 comprises a flow-chart 700 of the present method.

In step 702, an overall target volume is divided into sub-targets in accordance with the principles of spatially fractionated radiotherapy. The sub-targets will have characteristics such as location, size, shape or number, and prior to optimisation, these will have a set of initial values.

In step 704, an objective function or cost function is developed comprising dose-based cost terms that penalise deviation from clinical dosimetric goals. The dose-based cost terms may for example be based on clinical goals such as minimum dose to target, or maximum dose to organs at risk.

In step 706, one or more cost terms relating to sub-targets characteristics are included in the objective function developed in step 704.

In step 708, the objective function comprising the combination of dose-based cost terms and the sub-targets characteristics cost terms is iteratively optimised. The dose-based cost terms guide the optimisation process to provide treatment plans with optimal dose distribution in the sub-targets. The sub-targets characteristics cost terms guide the optimisation process to provide treatment plans having sub-targets with optimal characteristics, such as optimal locations, optimal shapes, optimal sizes or an optimal number.

In step 710, the plan optimiser checks whether certain optimisation stop criteria are satisfied. For example, the iterative optimisation may stop when the overall cost of the objective function optimised in step 708 is lower than a threshold value. In another example, the iterative optimisation may stop when a threshold number of iterations have been performed.

If at step 710 it is determined that the optimisation stop criteria are not satisfied, the method loops back to step 708 where the optimisation continues. If at step 710 it is determined that the optimisation stop criteria are satisfied, the method progresses to step 712.

In step 712, the treatment plan, optimised using an objective function comprising a combination of dose-based cost terms and sub-targets characteristics cost terms, is outputted. In particular, the outputted treatment plan may be used by a control unit to control a radiation treatment system in providing spatially fractionated radiotherapy according to the plan.

The method of Figure 7 does not have to be carried out in the particular sequence illustrated. For example, in some embodiments the sub-targets characteristics cost terms may be included in the objective function before the dose-based cost terms are included in the objective function, or both the sub-targets characteristics cost terms and the dose-based cost terms may be included in the objective function in a single step.

### Representing sub-targets location or shape

The location or shape of the sub-targets may be determined by defining an individual volume mask for each sub-target and updating the mask at every iteration during optimisation. For example, the model of the overall target volume may comprise a three-dimensional image, and the mask associated with each sub-target may be defined by labelling voxels of the three-dimensional image as being associated with the respective sub-target.

In other methods, the overall target volume may be represented as a single-distance-function where each voxel of the three-dimensional image is labelled with the shortest distance of the voxel to any target structure. A single-distance-function considers the union of multiple structures (here the union of all sub-targets) and defines a field where the value in a certain location in the field is the shortest distance to the surface of any sub-target structure. Numerically, the field can be defined as a regular lattice (and the distance is calculated at the lattice points) or an irregular mesh.

In another implementation, each voxel is additionally or alternatively labelled with an identification of the nearest target. This is used in a similar lattice/mesh to the distance field described above, by labelling every lattice point with an identifier of the target that is the closest target (when the closest distance to target surface is evaluated during the construction of the distance field, one could at the same time store to the same location the identifier of the nearest target). Such a lattice that provides the identification of the nearest target may be used in the computation of c^{dose-fit} when calculating the distance between the prescription dose isodose surface and the sub-target surface - to obtain the prescription dose, the identity of the target will be required. The identification of the nearest target in the lattice helps to distinguish between sub-targets, so that in the optimisation process, the correct cost term is updated.

The lattice points of the single distance function may be used to quickly calculate a distance between a lattice point and the boundary of the overall target volume, which is useful in determining the value of the cost term associated with the sub-target's distance to the boundary, or the value of the cost term associated with the "fit" of the sub-targets with the dose distribution. This avoids more complicated calculations of the boundary distance cost term c^{b} and the dosimetrical fit cost term c^{dose-fit}.

Thus, when optimising the location or shape of the sub-targets as defined above, the location or shape of the sub-targets may be defined by a volume mask or by the single-distance function. Other ways of representing sub-target shape or location are possible.

In summary, rather than defining sub-targets characteristics manually and separately from the dose-objective optimisation process, embodiments of the present invention generate the treatment plan by simultaneously optimising a combination of both (i) dose objectives and (ii) sub-target characteristics. This allows the sub-targets characteristics to be optimised in view of the desired dose distribution, such as the need to avoid irradiation of healthy tissue.

Those skilled in the art will recognise that a wide variety of modifications, alterations, and combinations can be made with respect to the above-described embodiments without departing from the scope of the invention, and that such modifications, alterations, and combinations are to be viewed as being within the ambit of the inventive concept.

## Claims

1. A method for generating and optimising a radiotherapy treatment plan for spatially fractionated radiotherapy, the method comprising:
constructing a model of patient anatomy, the model comprising a target volume and a plurality of sub-targets, wherein the sub-targets are determined by dividing the target volume;
and
generating the treatment plan by simultaneously optimising a combination of both (i) dose objectives and (ii) sub-targets characteristics.

2. **The** method of claim 1 wherein the sub-targets characteristics include any combination of one or more of:
(i) location of each of the sub-targets;
(ii) shape of each of the sub-targets;
(iii) size of each of the sub-targets; and
(iv) number of the sub-targets.

3. The method of claim 1 or claim 2 wherein the dose objectives comprise at least minimum dose to the target volume and/or maximum dose to an organ at risk.

4. The method of any preceding claim wherein the optimising comprises minimising or maximising the value of an objective function, wherein the objective function comprises at least one term related to the dose objectives, and at least one term related to the sub-targets characteristics.

5. The method of claim 4 wherein the objective function comprises at least one term related to the location of any of the sub-targets.

6. The method of claim 4 or claim 5 wherein the objective function includes at least one term related to a distance between a sub-target and a boundary of the target volume.

7. The method of any claim of 4 to claim 6 wherein the objective function includes at least one term related to an inter-target distance between two sub-targets in a plane normal to the beam axis;
and/or:
wherein the objective function includes at least one term related to an inter-target distance between two sub-targets in a direction parallel to a beam axis.

8. The method of any of claim 4 to claim 7 wherein the objective function includes at least one term related to how the sub-targets fit into a dose distribution comprising a prescription dose to targets and organs at risk.

9. The method of any of claim 4 to claim 8 wherein the objective function includes one or more of:
(a) at least one term related to the extent to which a prescription dose isodose surface conforms to a surface of a sub-target;
(b) at least one term that is an integral of square distances between points on a prescription dose isodose surface and points on a surface of a sub-target; and
(c) at least one term that is related to deviation of (i) a distance between a sub-target centre and a prescription dose isodose surface, from (ii) an average distance between the sub-target centre and the prescription dose isodose surface.

10. The method of any of claim 4 to claim 9 wherein the objective function comprises at least one term related to minimum dose for a target and/or maximum dose to an organ at risk.

11. The method of any preceding claim, wherein the optimising comprises one or more of:
(i) gradient based optimisation;
(ii) simulated annealing;
(iii) differential evolution; and
(iv) column generation.

12. The method of any preceding claim wherein the shape of one or more of the sub-targets is one or more of:
(i) spherical;
(ii) cylindrical; and
(iii) ellipsoid.

13. The method of any preceding claim comprising simultaneously optimising a combination of dose objectives, sub-target characteristics and any combination of:
fluence;
control points; and
multi-leaf collimator sequences;
of the treatment plan.

14. A radiation treatment planning computer system comprising a memory and a processor configured to perform the method as defined in any one of claims 1-13.

15. A computer program product comprising a non-transitory, computer readable storage medium encoded with instructions operable for execution by a processor to perform the method as defined in any one of claims 1-13.
